# EUROPEAN PATENT SPECIFICATION

(11) **EP 4 323 028 B1**
(45) Date of publication and mention of the grant of the patent: **01.07.2026**
(21) Application number: 21937120.0
(22) Date of filing: 26.12.2021
(51) Int. Cl.: A61L 27/06, A61L 27/12

(54) **HYBRID, ARTIFICIAL BONE TISSUE IMPLANT ABSORBING MECHANICAL VIBRATIONS, WHOSE ARCHITECTURAL STRUCTURE IMITATES TRABECULAR BONE, ALLOWING THE SATURATION OF BONE MARROW, BLOOD, AND NUTRIENTS, SUPPORTING AUTOLOGICAL REGENERATION, WHICH CAN BE USED WITH TITANIUM STRUCTURES**
HYBRIDES, KÜNSTLICHES KNOCHENGEWEBE-IMPLANTAT, DAS MECHANISCHE VIBRATIONEN ABSORBIERT, DESSEN ARCHITEKTONISCHE STRUKTUR TRABEKNOCHEN NACHAHMT, DIE SÄTTIGUNG VON KNOCHENMARK, BLUT UND NÄHRSTOFFEN ERMÖGLICHT UND DIE AUTOLOGISCHE REGENERATION UNTERSTÜTZT, DAS MIT TITANSTRUKTUREN VERWENDET WERDEN KANN
IMPLANT DE TISSU OSSEUX ARTIFICIEL HYBRIDE ABSORBANT LES VIBRATIONS MÉCANIQUES À STRUCTURE ARCHITECTURALE IMITANT L'OS TRABÉCULAIRE, PERMETTANT LA SATURATION DE LA MOELLE OSSEUSE, DU SANG ET DES NUTRIMENTS, FAVORISANT LA RÉGÉNÉRATION AUTOLOGUE, POUVANT ÊTRE UTILISÉ AVEC DES STRUCTURES EN TITANE

(30) Priority: 17.04.2021 WO PCT/TR2021/006756
(43) Date of publication of application: 21.02.2024
(73) Proprietor: Bloocell Saglik Teknolojileri Sanayi Ve Ticaret Limited Sirketi, Arifiye/Sakarya (TR)
(72) Inventor: HURI, Gazi, 54580 Çankaya/Ankara (TR); TOPALOGLU, Oktay, 54050 Serdivan/Sakarya (TR); ÇIKMAN, Mustafa Cüneyt, 41190 Basiskele/Kocaeli (TR)
(74) Representative: Kaya, Erdem
(86) International application number: PCT/TR2021/051516
(87) International publication number: WO 2022/220766

(56) References cited:
- WO-A1-2018/078130
- CN-A- 111 973 811
- CN-A- 112 590 203
- US-A1- 2019 009 004
- US-A1- 2020 397 542

## Description

### TECHNICAL FIELD

This invention relates to biological tissue implant comprising a composite scaffold formed by extrusion of polymer filaments and β-tricalcium phosphate onto a titanium mesh. The invention further relates to a method of producing such a scaffold, which includes generating micro-cracks via cryo-shock or vacuum drying and coating the scaffold with a physiological-buffered hyaluronic acid solution. The implant is designed to support cell infiltration and extracellular matrix interaction, providing a porous structure suitable for bone tissue regeneration.

### BACKGROUND

There are bioresorbable implants used in surgeries to repair various fractures such as foot fractures and to fill surgical defects. The bone marrow allows saturation with blood and nutrients with the developed architecture, thereby providing the patient's cells with the chemical signals needed for bone growth and remodeling. The meshwork or pores provide a rigid but flexible scaffold with adequate mechanical strength to support the growth of bone. As bone regeneration and remodeling takes place, it deteriorates. Long-term clinical trials showed that it provides significant bone regeneration because the materials are slowly absorbed by the body and replaced by autologous bone.

The tissue engineering field has advanced at a significant level in the last decade offering the potential to regenerate almost every tissue and organ of the human body. A typical tissue engineering strategy can be broken down into three components, which are *the scaffold, the cells,* and *the biological factors.* The scaffold serves as a template for tissue regeneration playing important roles in cell adhesion, proliferation, differentiation, and new tissue formation. The features expected from an ideal scaffold can be listed as follows, biocompatible and biodegradable structure with controllable degradation rates, decomposition products that will not cause inflammation and toxicity, a 3D and porous design to support cell adhesion, penetration, proliferation, and Extracellular Matrix (ECM (Extracellular matrix)) deposition, a network of interconnected pores to facilitate the passage of nutrients and waste, a suitable mechanical strength to support regeneration, and suitable surface chemistry and surface topography to promote cellular interactions and tissue development. Osteoconductivity is needed because of its porosity, and biodegradation, which are essential properties for scaffolds to be successful in bone tissue engineering applications, increase bone formation and angiogenesis and support osteoblast attachment and proliferation. Scaffolds can be produced from a variety of materials, including metals, ceramics, and polymers. Metallic alloys are used widely for dental and bone implants, and ceramics that have good osteoconductivity are used for bone tissue engineering. However, polymer materials are the dominant materials in the field of tissue engineering since metals are not biodegradable and cannot provide a matrix for cell growth and tissue formation. Ceramics also have limited biodegradability and cannot be processed as porous structures with their brittleness. Generally, polymer materials that are employed for scaffold production are immunologically advantageous as they are easy to process, biodegradable, and affect cell adhesion and function positively. For this reason, functionalized scaffolds can be produced that combine the advantage of both synthetic and natural polymeric materials by incorporating bioactive substances into synthetic polymers.

Polycaprolactone (PCL) is a fully biodegradable, thermoplastic polyester that has potential applications for bone and cartilage repair, and has been used successfully as a scaffold material in a variety of areas. PCL has thermal stability in the melted state with its positive features such as low glass transition temperature (60°C), low melting temperature (60°C), and high decomposition temperature (350°C). For this reason, semicrystalline PCL reaches a rubbery state at physiological temperature, and this results in high toughness and superior mechanical features (e.g. high strength and elasticity depending on its molecular weight) [9]. PCL degrades very slowly when compared to other biopolymers used in the body, and is suitable for use in long-term load-bearing applications with its high hydrophobicity and crystallinity. Various studies are conducted to produce PCL biocomposites with both natural and synthetic polymers and copolymers. PCL scaffolds can be produced with various rapid prototyping techniques e.g. FDM, SLS, low temperature, and multi-nozzle added manufacturing in which it was observed that the cells began to grow by adhering to the PCL scaffolds, and the feasibility of the produced scaffolds was demonstrated *in vitro* and *in vivo.* PCL, which is a synthetic biodegradable aliphatic polyester, is relatively inexpensive compared to other biomaterials, and its ability to mold into different forms makes it different from other biomaterials employed in scaffold development. PCL is an FDA-approved polyester and is suitable for both load-bearing and non-load-bearing tissue engineering applications. For this reason, it is also suitable for surface changes, its features such as hydrophobicity and degradation can be changed greatly. Recent advances in tissue engineering have led to the development of a scaffold that has ideal features by using composites or mixtures. With its hydrophobic nature, which affects the cell attractant properties of PCL, it is used in many experiments for blending with natural polymers, functionalizing its surface by using short amino acid stretches and peptide sequences such as fibrin. Adhesion, proliferation, and differentiation of seeded cells are enhanced by improving their biocompatibility.

Hyaluronic Acid (HA) is a glycosaminoglycan in extracellular tissue in many parts of the body as an increasingly important material for the science of biomaterials and finds applications in a wide variety of fields from tissue culture scaffolds to cosmetic materials. Its physical and biochemical features in solution or hydrogel form make it extremely attractive for technologies related to body repair. Since HA is rich in carboxyl and hydroxyl groups, it can form a hydrogel under conditions such as chemical modification, cross-linking, or photo-crosslinking. The mechanical strength and physical and chemical features of materials depend on the degree of modification and crosslinking. The purpose of cross-linking HA is to convert it from solid-state to hydrogel state under suitable conditions and prolong its residence time in the human body. Also, the mechanical strength of the cross-linked HA is higher at significant levels when compared to non-crosslinked ones, and makes it more suitable for tissue engineering applications. Cross-linked HA shows relatively higher mechanical features compared to its linear state. For this reason, its use as a composite may combine the advantages of different materials.

The technique that is employed to produce PCL scaffolds depends on the type of scaffold required. Methods such as 3D printing, phase separation technique, and freeze-drying are used for porous scaffolds. However, techniques e.g. electrospinning are also used to produce fibrous scaffolds. Features such as pore structure, pore size, hardness, and permeability require precise process control. For this reason, 3D printing technology can overcome many of the limitations of traditional manufacturing techniques offering ease of control of production parameters, versatile pore geometry, 100% pore connectivity, and repeatability.

In the patent document showing the state of the art with the number of 2018/11205 and with the title "Osteogenic osteoconductive biocompatible composite nanofiber scaffold for bone and cartilage tissue damage repair" it is stated that it is made of Polycaprolactone (PCL), bovine gelatin (GE) and Bovine Hydroxyapatite (BHA) to be used in bone and cartilage tissue damage repair applications as a biocompatible, osteogenic, osteoconductive biomimetic composite nanofiber scaffold produced with the electrospinning method and which are selected because of their high biocompatibility, chemical properties, and similarity to bone, with nontoxic solvent system of these materials, and the parameters of the composite nanofiber production process by electrospinning method are described along with the physical, morphological, chemical, mechanical, and biological properties of PCL/GE/BHA nanofibers, and with the statistical significance of cell proliferation and viability test results, composite nanofiber scaffolds produced by osteoblast biocompatible with human cells.

In the patent with the document number of 2019/12510 and with the title "A composite biomaterial suitable to be used in bone tissue repair", which shows the state of the art, including the steps of boron and polylactic acid-containing composite biomaterials suitable for use in bone tissue repair and their production, weighing and melting the polylactic acid powder, adding and mixing boron powder into molten polylactic acid, cooling the polylactic acid and boron mixture to become pellets, producing polylactic acid and boron-containing filaments by extruding the pellets, and producing composite biomaterials by processing the filaments.

In the patent document with the number of 2016/18844 and wit the title "Silver-ion added calcium phosphate-based bioceramic artificial bone tissue with antimicrobial properties", which shows the state of the art, an artificial bone tissue material, which can be used for the treatment of osteomyelitis and implant-related bone infections in humans and animals is produced the most important feature of which is also to treat bone infections which result in cavities, as a biocompatible artificial bone tissue material with anti-microbial properties obtained by applying nano-technological approaches, with the basic structure of calcium phosphate with silver ion added with the Wet Chemical Method used in the production of silver-added antimicrobial bioceramics, the powders are Biphasic (HAP+TCP) and Triphasic (HAP+TCP+Bioglass), silver ions are added to the structure, giving antimicrobial properties, the synthesized nanopowder and the material that will form the porous structure mixed in the desired amounts as an artificial bone tissue material. Another study, CN112590203 A, relates to a controllable gradient scaffold loaded with drugs, biological active factors, and cells, produced using a multi-nozzle 3D printer. In this invention, a multilayer structure with functional gradients is obtained by alternating the deposition of thermoplastic and hydrogel-based materials. While the document discloses the use of known biomaterials such as β-tricalcium phosphate (β-TCP) and polycaprolactone (PCL), it does not mention the use of a titanium mesh, the formation of micro-cracks by cryo-shock or vacuum drying, or a scaffold porosity specifically in the range of 50-70 microns to support extracellular matrix interaction and mechanical performance.

### Aims of the Invention:

With our invention, the purpose is to increase the transmission rate of the growth factors of the 3D polymer and p-Tricalcium Phosphate (p-TCP) scaffold structure formed here.

Another purpose of the invention was to obtain a hybrid artificial bone tissue implant absorbing mechanical vibrations, which can be used with titanium structures supporting autologous regeneration, allowing the saturation of bone marrow, blood, and nutrients with an architectural structure imitating trabecular bone.

Another purpose of the invention was to obtain a 3D and porous design to support cell adhesion, penetration, proliferation, and extracellular matrix (ECM (Extracellular Matrix)) deposition.

Another purpose of the invention was to reinforce bone tissues that cannot be shaped or volumized again.

Another purpose of the invention was to increase the growth factors by providing cell adhesion with HA coating with scaffold production and 3D printing technique.

### BRIEF DESCRIPTION OF THE DRAWINGS

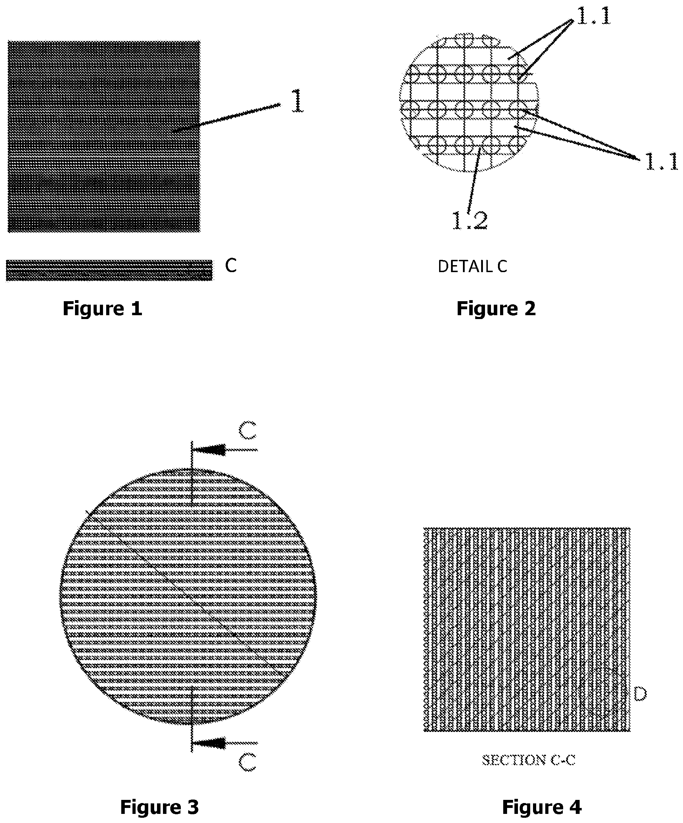

- Figure 1:: The schematic view of the cylindrical tissue scaffold.
- Figure 2:: Detail view of the cylindrical tissue scaffold.
- Figure 3:: The schematic view of the front face of the cylindrical scaffold.
- Figure 4:: The schematic view of the cartilage repair patch.

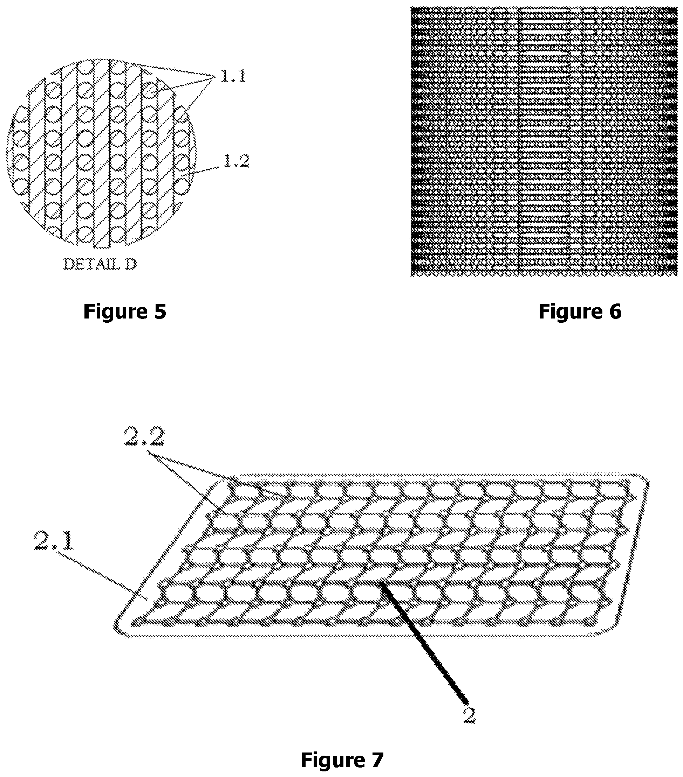

- Figure 5:: The schematic and detailed view of the filament structure.
- Figure 6:: The schematic view of the filament array (cross).
- Figure 7:: The schematic-perspective view of the hybrid system.

The parts in the figures are numbered one by one and are given below:
1. Tissue scaffold
   1.1. Filaments
   1.2. Chamber
2. Hybrid implant
   2.1. Polymer layer
   2.2. Titanium mesh

### DETAILED DESCRIPTIN OF THE INVENTION

In this detailed description, the invention is basically the creation of a 3D polymer and β-Tricalcium Phosphate (β-TCP) scaffold structure (tissue scaffold) and a biological tissue implant allowing/supporting cell infiltration by using the added manufacturing process, coated with physiological-buffered HA solution with the deep coting method to increase the delivery rate of growth factors and enlarge their areas allowing the use of titanium mesh plate.

Since a normal hyaluronic acid molecule is metabolized and excreted 12 hours after it is injected into the human skin, cross-links are used in HA molecules to make it permanent. The cross-linking of hyaluronic acid makes the solution more viscous increasing its effect by prolonging the residence time in the implant.

The layers are connected at angles to support extracellular matrix (ECM) interaction in our invention, and the overlapping structures are overlapped obliquely. The resulting structures have an hfa 50-70 micron porous structure and support the formation of vascularized tissue with osteoconductive effect.

The 3D tissue scaffold formed as a result of extrusion crates micro-cracks on the body with the cryo-shocking method, and the deep encapsulation of hyaluronic acid into the body is increased. The HA solution is 20-70 µm-1 mL per square centimeter of scaffold body.

It is a biological tissue implant and its features are;
- Overlapping filament layers (1.1) connected at angles to support Extracellular Matrix (ECM) interaction with each other connected by angling at 90° to each other,
- Oblique overlapping structures of the third filament layer,
- Supporting the formation of vascularized tissue with the osteoconductive effects of 50-70-micron pore structure of the obtained structures,
- Increasing the encapsulation of the hyaluronic acid deep into the body by creating micro cracks with the cryo-shock method or vacuum drying system of the 3D tissue scaffold (1) formed as a result of extrusion,
- Attachment and coating of empass or hot polymer (2.1) onto the surface of the titanium mesh (2.2) by extrusion,

Bone augmentation (to patients with a bone deficiency) to repair severely traumatic and degraded tissues is not suitable for reshaping especially in the jaw region if the discomfort has gained aesthetic concern. It will be an important solution for bone tissue that cannot be reshaped or volumized. It should be noted that methods of bone augmentation or repair are not according to the invention as claimed.

Another feature of the system is the polymer implant technology that has a hybrid structure. Bionic titanium, which is the raw material of the scaffold obtained, is coated on the mesh plate with the extrusion or drying method. The vascularized tissue is re-grown and formed inward by reshaping the volumetric defect. The tissue is protected against environmental loads while shaped with a titanium mesh scaffold thanks to this technology. The reinforced titanium plates provide a barrier by minimizing softness. The titanium plates are, however, not according to the invention as claimed. The titanium mesh, which is part of the invention claimed, also provides radiographic visibility. It is of vital importance especially for the defects in the head region with its high ability to imitate bone.

The hybrid structure (2) fully supports bone augmentation, acts as a barrier, has high-density polymer tissue, helps in the regeneration, provides potential fibrovascular growth, covers the polymer structure (2.1) on titanium mesh (2.2), and provides form and volume to the tissue, which has lost its volumetric integrity, and has the feature allowing the tissue to grow inward. The thin polymer layer (2.1) on the implants minimizes the upper surface tissue adhesion supporting vascular tissue growth by increasing the lower surface porous structure.

A second outer cell has a permeable HA layer and a cartilagenic matrix (architecture) between the first and second layers. The cartilagenic matrix and the permeate layer surface area have the characteristics of a receiving point for the diffusion of autologous stem cells and has components supporting the production of hyaline-like cartilage in the presence of autologous stem cells.

The accurate combination of nano-enhancer and hydrogel polymer, hyaluronic acid coating method to produce mechanically firm, electrically conductive, bioactive;

It contains the following process steps;
- The preparation of solutions with a magnetic stirrer at room temperature with 10mg/ml sodium hyaluronate (~ 1 million Da, medical-grade) in physiological buffer (PBS pH 7.4),
- Completing the work by coating the scaffolds with dip-coating method into the solution and drying at vacuum oven at 50°C for 3 days,

Although the biological tissue implant described herein may be adaptable to various geometrical configurations, such as cylindrical, square, or anatomically contoured shapes, these variations are not essential to the core of the claimed invention. Likewise, potential design elements such as fixation holes and radial titanium mesh configurations Therefore, these embodiments are disclosed solely as illustrative examples and are not intended to limit the claimed invention in any way.

If desired, therapeutic concentration, stem cell, or growth factor can be integrated into the scaffold structure.

β-Tricalcium Phosphate (β-TCP) is included in the prepared PCL granule by 3-15%. In this way, the toughness values of the scaffold body formed by reducing the viscosity of the polymer are increased.

β-Tricalcium Phosphate (β-TCP) is a biocompatible, radiopaque, and resorbable osteoconductive material as an important factor supporting the formation of new bone in the defect area.

## Claims

1. A biological tissue implant, wherein it comprises:
- a titanium mesh,
- a composite of polymer filaments and beta-tricalcium phosphate, which have been extruded onto the surface of the titanium mesh, the composite being coated with hyaluronic acid and containing micro-cracks;
- the resulting pore structure is in the range of 50 to 70 microns,
- the polymer filaments in the composite are overlapped to form an angle of 90° to support extracellular matrix interaction and with oblique overlapping structures of the third filament layer,
- the scaffold comprises micro-cracks formed by cryo-shock or vacuum drying,
- and the composite structure is coated with a physiological-buffered hyaluronic acid solution.

2. The biological tissue implant according to one of the preceding claims, wherein it comprises at least one of the group of therapeutic concentration, stem cell, growth factor.

3. A method for obtaining a biological tissue implant, **characterized in that** it comprises the following process steps:
- obtaining a titanium mesh plate,
- attaching beta-tricalcium phosphate-containing polymer filaments onto the surface of the titanium mesh by extrusion,
- arranging the polymer filaments in overlapping layers at an angle of 90°,
- adding a third filament layer with obliquely overlapping structures to form a 3D tissue scaffold,
- applying at least one of the cryo-shocking or vacuum drying methods to the 3D scaffold formed by extrusion, thereby creating micro-cracks,
- coating the tissue scaffold with physiological-buffered hyaluronic acid solution by dip-coating method.

4. The method according to claim 3, **characterized in that** the dip-coting method with hyaluronic acid comprises the following process steps:
- adding 10 mg/ml of sodium hyaluronate in PBS and obtaining a mixture with a pH value of 7.4,
- dipping the obtained tissue scaffold in the resulting mixture,
- drying at 50°C for 3 days by means of a vacuum oven.

5. The method according to claim 4, wherein sodium hyaluronate is medical grade sodium hyaluronate and has a molecular weight of 1 million Da.

## Patentansprüche

1. Biologisches Gewebeimplantat, wobei es umfasst:
- ein Titangitter,
- einen Verbund aus Polymerfilamenten und Beta-Tricalciumphosphat, die auf die Oberfläche des Titangitters extrudiert wurden, wobei der Verbund mit Hyaluronsäure beschichtet ist und Mikrorisse aufweist;
- die resultierende Porenstruktur liegt im Bereich von 50 bis 70 Mikrometern,
- die Polymerfilamente im Verbund sind in einem Winkel von 90° überlappend angeordnet, um die Interaktion mit der extrazellulären Matrix zu fördern, und weisen schräge Überlappungsstrukturen der dritten Filamentschicht auf,
- das Gerüst weist Mikrorisse auf, die durch Kryoschock oder Vakuumtrocknung entstanden sind,
- und die Verbundstruktur ist mit einer physiologisch gepufferten Hyaluronsäurelösung beschichtet.

2. Biologisches Gewebeimplantat nach einem der vorstehenden Ansprüche, wobei es mindestens eines der folgenden Elemente umfasst: therapeutische Konzentration, Stammzellen, Wachstumsfaktoren.

3. Verfahren zur Herstellung eines biologischen Gewebeimplantats, **dadurch gekennzeichnet, dass** es die folgenden Verfahrensschritte umfasst:
- Bereitstellen einer Titan-Gitterplatte,
- Aufbringen von Beta-Tricalciumphosphat-haltigen Polymerfilamenten auf die Oberfläche des Titangitters durch Extrusion,
- Anordnen der Polymerfilamente in überlappenden Schichten in einem Winkel von 90°,
- Hinzufügen einer dritten Filamentschicht mit schräg überlappenden Strukturen zur Bildung eines 3D-Gewebegerüsts,
- Anwenden mindestens eines der Verfahren des Kryoschock oder der Vakuumtrocknung auf das durch Extrusion geformte 3D-Gerüst, wodurch Mikrorisse erzeugt werden,
- Beschichten des Gewebegerüsts mit einer physiologisch gepufferten Hyaluronsäurelösung im Tauchbeschichtungsverfahren.

4. Verfahren nach Anspruch 3, **dadurch gekennzeichnet, dass** das Tauchbeschichtungsverfahren mit Hyaluronsäure die folgenden Verfahrensschritte umfasst:
- Hinzufügen von 10 mg/ml Natriumhyaluronat in PBS und Erhalten einer Mischung mit einem pH-Wert von 7,4,
- Eintauchen des erhaltenen Gewebegerüsts in die resultierende Mischung,
- Trocknen bei 50°C über einen Zeitraum von 3 Tagen mittels eines Vakuumofens.

5. Verfahren nach Anspruch 4, wobei es sich bei dem Natriumhyaluronat um medizinisches Natriumhyaluronat handelt und dieses ein Molekulargewicht von 1 Million Da aufweist.

## Revendications

1. Implant de tissu biologique, dans lequel il comprend :
- un treillis en titane,
- un composite constitué de filaments polymères et de phosphate tricalcique bêta, qui ont été extrudés sur la surface du treillis en titane, ledit composite étant enduit d'acide hyaluronique et comprenant des microfissures ;
- la structure poreuse obtenue se situe dans une gamme de 50 à 70 microns,
- les filaments polymères du composite sont superposés de manière à former un angle de 90° afin de favoriser l'interaction avec la matrice extracellulaire et avec des structures obliques superposées de la troisième couche de filaments,
- l'échafaudage comprend des microfissures formées par choc cryogénique ou par séchage sous vide,
- et la structure composite est enduite d'une solution d'acide hyaluronique tamponnée physiologiquement.

2. Implant de tissu biologique selon l'une des revendications précédentes, dans lequel il comprend au moins un du groupe concentration thérapeutique, cellule souche, facteur de croissance.

3. Méthode permettant d'obtenir un implant de tissu biologique, **caractérisé en ce qu'**elle comprend les étapes du procédé suivantes :
- obtenir une plaque de treillis en titane,
- fixer, par extrusion, des filaments de polymère contenant du phosphate tricalcique bêta à la surface du treillis en titane,
- disposer les filaments de polymère en couches superposées à un angle de 90°,
- ajouter une troisième couche de filaments comportant des structures obliques superposées afin de former une échafaudage tissulaire en 3D,
- appliquer au moins l'une des méthodes de choc cryogénique ou de séchage sous vide à l'échafaudage en 3D formée par extrusion, créant ainsi des microfissures,
- enduire l'échafaudage tissulaire d'une solution d'acide hyaluronique tamponnée physiologiquement par la méthode d'enduction par immersion.

4. Méthode selon la revendication 3, **caractérisée en ce que** la méthode d'enduction par immersion à l'acide hyaluronique comprend les étapes du procédé suivantes :
- ajouter 10 mg/ml d'hyaluronate de sodium dans du PBS et obtenir un mélange dont le pH est de 7,4,
- immerger l'échafaudage tissulaire obtenue dans le mélange résultant,
- sécher à 50 °C pendant 3 jours au moyen d'un four à vide.

5. Méthode selon la revendication 4, dans laquelle l'hyaluronate de sodium est un hyaluronate de sodium de qualité médicale et présente un poids moléculaire de 1 million de Da.
